# EUROPEAN PATENT SPECIFICATION

(11) **EP 1 345 559 B1**
(45) Date of publication and mention of the grant of the patent: **10.01.2007**
(21) Application number: 01930313.0
(22) Date of filing: 07.05.2001
(51) Int. Cl.: A61F 5/11, A61B 17/54

(54) **PEDICURE TOOL FOR CLEANING SKIN TISSUES**
FUSSPFLEGEWERKZEUG ZUM REINIGEN VON HAUTGEWEBE
INSTRUMENT DE PEDICURE PERMETTANT DE NETTOYER DES TISSUS CUTANES

(30) Priority: 20.12.2000 NL 1016926
(43) Date of publication of application: 24.09.2003
(73) Proprietor: Spapens, Irene, 2134 CP Hoofddorp (NL)
(72) Inventor: Spapens, Irene, 2134 CP Hoofddorp (NL)
(86) International application number: PCT/NL2001/000346
(87) International publication number: WO 2002/049550

(56) References cited:
- US-A- 2 202 926
- US-A- 2 499 851
- US-A- 2 542 324
- US-A- 4 621 619
- US-A- 5 437 679
- US-A- 5 645 554

## Description

The invention relates to a pedicure tool for cleaning skin tissue which is present under the nail of a human toe according to the preamble of claim 1.

From US-A-2,542,324, on which the preamble of claim 1 is based, a device is known for relieving and correcting ingrown toe nails. It is a strip of flexible metal, one end of which is turned back on itself to engage between the side edge of the toe nail and the adjacent skin. This known device has only limited length and in use extends only aside the toe to which it is applied. It is attached to the toe by an adhesive strip such that it can be kept in place while wearing shoes. US-A-2,499,851 discloses similar devices. US-A-4,621,619 discloses a retractor, the shape of which is unsuited as a pedicure tool as contemplated here.

The object of the invention is to provide a pedicure tool of the kind referred to in the introduction, which creates sufficient space for the cutting tool in an efficient and simple manner, and which moreover provides sufficient support for the pedicure to enable him or her to handle the cutter in an accurate manner, thus reducing the risk of healthy tissue being damaged.

In order to accomplish that objective, the pedicure tool is as according to claim 1. The sub claims disclose prefered embodiments.

In addition to that, the present pedicure tool protects the other toe tissue from being damaged undesirably by treatment tools. Furthermore, the tool functions as a support for the pedicure's fingers.

The hook preferably makes an angle of 80 - 150° with respect to the clamping means that are preferably fitted at least partially round the toe. The hook can thus form an acute angle or a right angle, but under certain circumstances an obtuse angle may be preferred, depending on the shape of the toe and the treatment the toe is to undergo.

Preferably, the pulling means, for example said hook, have a pointed end, so that it can easily be moved between the nail edge and the skin fold.

The invention will now be explained in more detail by means of an exemplary embodiment as shown in the figures, wherein:
Figure 1 is a perspective view of a pedicure tool;
Figure 2 is a perspective view of a toe around which the pedicure tool of Figure 1 is fitted; and
Figure 3 is a schematic, perspective view of the use of the pedicure tool of Figure 1.

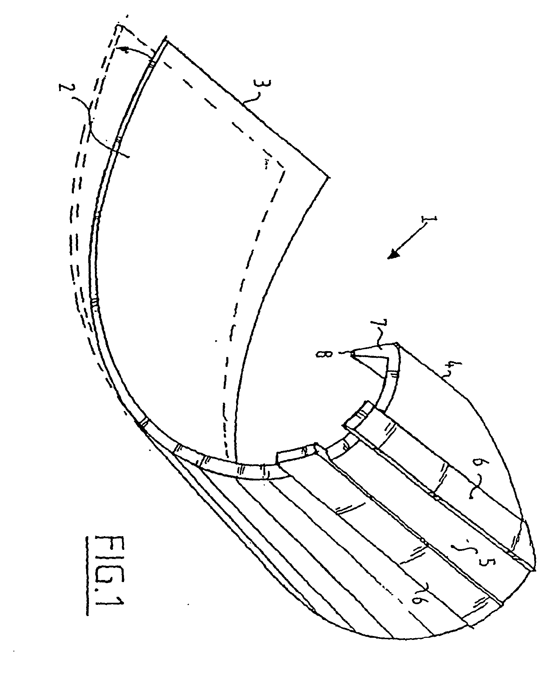

The plastic pedicure tool as shown in Figure 1 is made in one piece of, for example, polypropylene, using an injection moulding process. Tool 1 comprises a flexible clamping portion 2, which mainly consists of a curved strip of material, whereby the thickness of the material decreases towards end 3, as a result of which the material becomes more flexible in that direction, a characteristic which is schematically illustrated by the broken lines.

This makes it readily possible to clamp the tool 1 around toes of varying dimensions. Near the other end 4 of the tool, said strip of material comprises a contacting portion 5 substantially consisting of two ribs 6, which project upwards from the strip-shaped material. Ribs 6 extend on either side of the strip, as a result of which an additional contacting possibility is created. Also ribs of different shapes and orientations are conceivable and possible. A hook-shaped, inwardly extending projection 7 is formed on end 4, which projection forms pulling means for pulling the skin fold of a toe away from the abutting nail. Projection 7 tapers off into a rounded, sharp point 8, so that it can be easily inserted between the nail and the skin fold. Furthermore, the width of projection 7 is smaller than the width of clamping portion 2 in order to facilitate insertion thereof. Since the material of tool 1 is relatively thick near the end 4, tool 1 is stiff and inflexible on this side, so that the tool 1 is capable of transmitting a sufficiently large force onto the aforesaid skin fold without being deformed thereby.

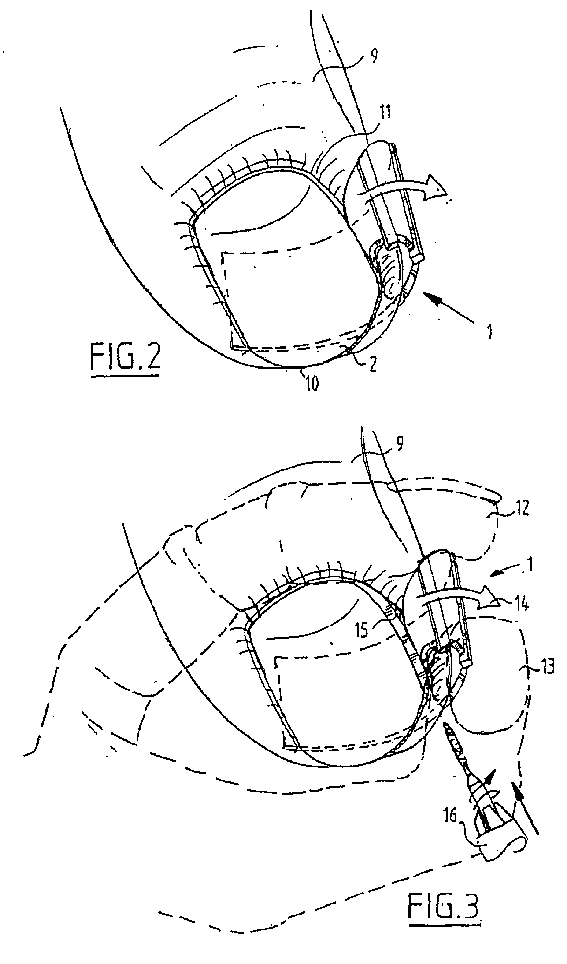

Figure 2 shows the manner in which tool 1 is fitted round a toe 9. The flexible clamping portion 2 is slightly straightened and placed round toe 9, so that the elasticity of the tool 1 will cause the tool to clamp itself down. Then the hook-shaped projection 7 is inserted between the nail edge 10 and the skin fold 11.

Figure 3 shows how the contacting portion 5 of tool 1, and in particular the ribs 6 thereof, can be taken hold of by a pedicure with his or her index finger 12 and thumb 13. By subsequently exerting a pushing force in the direction of arrow 14, a space 15 is created between the toe nail and the adjacent and underlying toe tissue. The pedicure can then easily clean said tissue with a rotating cutter 16 and remove any inflamed tissue whilst fingers 13, 14 rest on the tool. Fingers 13, 14 can in turn provide support for the pedicure's right hand which handles the cutter 16.

The embodiment of the tool that is shown herein is not symmetrical, and consequently it can be produced in a left-hand version as well as in a right-hand version. Alternatively, the pointed projection 7 can be positioned in the centre of the strip-shaped material, thus enabling two-sided use of the tool 1.

## Claims

1. A pedicure tool (1) for cleaning skin tissue which is present under the nail (2) of a human toe (9), comprising pulling means (7) which can be placed in abutment with a skin fold (11) abutting against the nail edge (10) wherein said pulling means (7) comprise a hook-shaped portion, to engage said skin fold (11), and contacting means (5, 6), on which a manual force can be exerted in order to pull back the skin fold (11) from the nail edge (10), using.said pulling means (7), in such a manner that the skin tissue to be treated will be exposed, **characterised in that** it further comprises clamping means (2) by which the tool (1) can be clamped round the toe (9), extending said tool by an elastically deformable, bent strip dimensioned and shaped to extend from aside a toe to below said toe and engage the underside of said toe such that said tool is adapted to clamp said toe between said hooked shaped portion engaging said skin fold and said bent strip engaging the underside of said toe.

2. A pedicure tool (1) according to claim 1, wherein said pulling means (7) have a pointed end (8).

3. A pedicure tool (1) according to claim 1 or 2, wherein thickness of the strip (2) decreases from a largest thickness near the pulling means (7) to a smallest thickness near the end (4) of the strip (2) that is positioned opposite the pulling means.

4. A pedicure tool (1) according to claim 1, 2 or 3, wherein said contacting means (5, 6) comprise at least one rib (6), which extends in transverse direction to the strip (2).

5. A pedicure tool (1) according to claim 1, 2, 3 or 4, wherein said contacting means (5, 6) extend laterally with respect to the strip (2).

6. A pedicure tool (1) according to any one of the preceding claims 1-5, wherein said tool (1) is substantially made in one piece.

7. A pedicure tool (1) according to any one of the preceding claims 1-6, wherein said tool (1) is made of plastic material.

8. A pedicure tool (1) according to any of the preceding claims 1-7, wherein said contacting means (5, 6) comprise at least one bead (6) projecting from the tool near said hook shaped portion.

9. A pedicure tool (1) according to any of the preceding claims 1-8, wherein said contacting means (5, 6) are provided such that when installed at a toe nail the pedicure can place her thumb or indexing finger against said means (5, 6) when gripping said tool between her indexing finger and thumb and pulling at said skin fold (11) by said tool, such that said contacting means improve the grip of the fingers.

10. A pedicure tool (1) according to any of the preceding claims 1-8, wherein said hook-shaped portion makes an angle between 80° and 150° relative to said clamping means.

## Patentansprüche

1. Ein Fußpflegewerkzeug (1) zur Entfernung von Hautgewebe unter den Zehen (2) des menschlichen Fußes (9), bestehend aus einem Klemm-Mechanismus (2), mit welchem das Werkzeug (1) am Zeh (9) festgeklemmt werden kann, einem Ziehmechanismus (7), der an einer Hautfalte (11) anliegend positioniert werden kann und dabei an der Nagelkante (10) anliegt, sowie Berührungselemente (5, 6), auf die von Hand eine Kraft ausgeübt werden kann, um die Hautfalte (11) von der Nagelkante (10) mit Hilfe des besagten Ziehmechanismus (7) zurück zu ziehen, und zwar in einer Weise, dass das zu behandelnde Hautgewebe freigesetzt wird das sich dadurch auszeichnet, dass es ebenfalls besteht aus, wobei der Ziehmechanismus einen rechteckig geformten Abschnitt umfasst, mit dem die besagte Hautfalte (11) umschlossen werden kann, wodurch das besagte Werkzeug durch einen elastisch verformbaren Biegestreifen erweitert wird, der so geformt ist, dass er von der Zehenseite bis unter den besagten Zeh verläuft und die Unterseite des besagten Zehs derart umschließt, dass das besagte Werkzeug den besagten Zeh in den besagten rechteckigen Abschnitt klemmen kann, wobei die besagte Hautfalte und der besagte Biegestreifen sowie die Unterseite des Zehs unschlossen werden.

2. Ein Fußpflegewerkzeug (1) gemäß Antrag 1, in dem der besagte Ziehmechanismus (7) ein spitzes Ende (8) hat.

3. Ein Fußpflegewerkzeug (1) gemäß Antrag 1 oder 2, bei dem die Dicke des Streifens (2) vom Maximum beim Zugmechanismus (7) bis zum Minimum hin am Ende (4) des Streifens (2), welches gegenüber dem Zugmechanismus positioniert ist, abnimmt.

4. Ein Fußpflegewerkzeug (1) gemäß Antrag 1, 2 oder 3, bei dem die besagten Berührungselemente (5, 6) mindestens eine Lamelle (6) umfassen, die diagonal zum Streifen (2) verläuft.

5. Ein Fußpflegewerkzeug (1) gemäß Antrag 1, 2, 3 oder 4, bei dem die besagten Berührungselemente (5, 6) seitlich zum Streifen (2) stehen.

6. Ein Fußpflegewerkzeug (1) gemäß eines der obigen Anträge 1-5, bei dem das besagte Werkzeug (1) grundsätzlich aus einem Teil besteht.

7. Ein Fußpflegewerkzeug (1) gemäß eines der obigen Anträge 1-6, bei dem das besagte Werkzeug (1) aus Plastik hergestellt wird.

8. Ein Fußpflegewerkzeug (1) gemäß eines der obigen Anträge 1-7, bei dem die besagten Berührungselemente (5, 6) mindestens eine Perle (6) umfassen, die nahe des rechteckigen Ausschnitts aus dem Werkzeug herausragt.

9. Ein Fußpflegewerkzeug gemäß eines der obigen Anträge 1-8, bei welchem die besagten Berührungselemente (5, 6) so ausgestattet sind, dass die Fußpflegerin bei Positionierung des Werkzeugs an einen Zehennagel ihren Daumen oder Zeigefinger auf die besagten Elemente legen kann, wenn sie das besagte Werkzeug zwischen Daumen und Zeigefinger greift und an der besagten Hautfalte (11) mit dem besagten Werkzeug zieht, so dass die besagten Berührungselemente die Fingergriffigkeit verbessern.

10. Ein Fußpflegewerkzeug (1) gemäß eines der obigen Anträge 1-8, bei dem der rechteckige Abschnitt einen Winkel von 80° bis 150° im Verhältnis zum besagten Klemm-Mechanismus vollzieht.

## Revendications

1. un instrument de pédicurie pour (1) nettoyer le tissue cutané se trouvant sous l'ongle (2) d'un orteil (9), comprenant [un dispositif de mâchoire (2) par lequel l'instrument (1) peut être serré sur l'orteil (9)], un dispositif de traction (7) qui peut être placé par butée sur un pli cutané (11) en buté contre le bord de l'ongle (10), et un dispositif de contact (5, 6), sur lequel une force manuelle peut être exercée de façon à tirer sur le pli cutané (11) à partir du bord de l'ongle (10), en utilisant le dispositif de traction (7), de telle façon à ce que le tissu cutané devant être soigné, soit exposé, **caractérisée par le fait qu'**il comprend en plus le dispositif de traction en question (7) comprend une extrémité en forme de crochet, à appliquer sur le pli cutané (11)] une extension de l'instrument en question constituée d'une lamelle en plastique déforme le courbée conçue et taillée pour s'étendre du côté de l'orteil jusqu'au-dessous de l'orteil en question et s'engager sous la face de l'orteil de manière à ce que l'instrument en question s' adapte pour serrer l'orteil entre l'extrémité crochetée s'appliquant sur le pli cutané et la lamelle courbée s'appliquant en dessous de l'orteil.

2. Un instrument de pédicurie (i) conformément à la requête 1 ou le dispositif de traction en question (7) comporte une extrémité en pointe (8).

3. Un instrument de pédicurie (1) conformément à la requête 1 ou 2, où l'épaisseur de la lamelle (2) s'affine à partir de l'extrémité la plus large près du dispositif de traction (7) jusqu'à atteindre une épaisseur moindre près de l'extrémité (4) de la lamelle (2) située à l'opposé du dispositif de traction.

4. Un instrument de pédicurie (1) conformément à la requête 1, 2,ou 3 où le dispositif de contact en question (5, 6) comprend au moins une arrête ( ), qui s'étend en transversale vers la lamelle (2).

5. Un instrument de pédicurie (1) conformément à la requête 1, 2, 3, 4 ou 5 où le dispositif de contact (5, 6) s'étend de manière latérale par rapport à la lamelle (2).

6. Un instrument de pédicurie (1) conformément à toutes les requêtes 1 - 5 précédentes, où l'instrument en question (1) est constitué d'une seule pièce.

7. Un instrument de pédicurie (1) conformément à toutes les requêtes 1 - 6 précédentes, où l'instrument en question (1) est en plastique.

8. un instrument de pédicurie (1) conformément à toutes les requêtes 1-7 précédente, ou le dispositif de contact en question (5,6) comprend au moins une nervure (6) en avant de l'extrémité de l'instrument en crochet.

9. un instrument de pédicurie (1) conformément à toutes les requêtes 1-8 précédentes, où les dispositifs de contact en question (5, 6) sont fournis de manière à ce que lorsqu'il est installé sur l'ongle d'un orteil, la pédicure puisse placer son pouce ou son index contre ce dispositif (5, 6) lorsqu'elle tient l'instrument en question entre son index et son pouce, et tire sur le pli cutané (11) grâce à instrument en question, de façon à ce que le dispositif de contact en question améliore la préhension de ses doigts.

10. un instrument de pédicurie (1) conformément à toutes les requêtes 1-8 précédentes, où la parti en forme de crochet présente un angle entre 80° et 150° par rapport au dispositif de serrage.
